# EUROPEAN PATENT SPECIFICATION

(11) **EP 4 585 233 B1**
(45) Date of publication and mention of the grant of the patent: **07.10.2026**
(21) Application number: 24197600.0
(22) Date of filing: 30.08.2024
(51) Int. Cl.: A61L 27/36, A61L 27/38, A61L 27/54, A61L 27/60, A61L 15/40, A61L 15/44

(54) **COMPOSITION COMPRISING FIBROUS ACELLULAR DERMAL MATRIX AND METHOD FOR PREPARING SAME**
ZUSAMMENSETZUNG ENTHALTEND EINE FASERIGE AZELLULÄRE HAUTMATRIX
COMPOSITION COMPRENANT UNE MATRICE DERMIQUE ACELLULAIRE FIBREUSE ET SON PROCÉDÉ DE PRÉPARATION

(30) Priority: 10.01.2024 KR 20240003879; 02.02.2024 KR 20240016543
(43) Date of publication of application: 16.07.2025
(73) Proprietor: CGBIO Co.,Ltd., Seoul 04349 (KR)
(72) Inventor: WOO, Jiwon, Bucheon-si, Gyeonggi-do 14417 (KR); LEE, Kwon Young, Seongnam-si Gyeonggi-do 13172 (KR); YOON, Hong Sun, Yongin-si, Gyeonggi-do 17117 (KR)
(74) Representative: Viering, Jentschura & Partner mbB Patent- und Rechtsanwälte

(56) References cited:
- KR-A- 20230 008 422
- US-A1- 2010 272 782

## Description

### [Technical Field]

The present disclosure relates to a composition comprising a fibrous acellular dermal matrix and a method for preparing the same, and more particularly, to a composition comprising a drying method-applied fibrous acellular dermal matrix and a method for preparing the same.

### [Background Art]

A wound is a condition in which the continuity of the tissue is destroyed by the rupture or defect of the skin or other tissue due to external pressure, and usually refers to a phenomenon in which the skin is opened by damaging the dermis layer of the skin. Wound surgery basically prevents infection and suppresses inflammatory reactions by suturing and dressing the wound site to block it from being exposed to the external environment. The materials for wound dressings can be largely divided into allogeneic dermis and xenogeneic dermis, and the wound dressings can be manufactured by a method of extracting specific polymers from the materials and a method of using a dermal matrix.

Acellular dermal matrix (ADM) is one in which the cells in the dermis are removed after removing the epidermis from the skin tissue of a donated cadaver in order to eliminate immune rejection reactions, and it is widely used in soft tissue reconstruction in this field and is also widely used in allograft for burn treatment. The dermal tissue is composed of 80 to 90% collagen, elastin, and glycosaminoglycan.

Meanwhile, in 1994, LifeCell prepared an acellular dermal matrix (ADM) by decellularizing and freeze-drying skin tissue collected from cadavers and commercialized it as Alloderm (allograft) for use in burn treatment, skin reconstruction, etc. Alloderm (allograft) was safer than heterogeneous products and showed much better survival rate and healing effect. As similar products like this, Bard Dabol's AlloMax, Ethicon's FlexHD, etc. were developed. However, the products described as above had the problem of having poor flexibility, making them difficult to use on curved or deep wounded areas.

To solve such a problem, LifeCell developed a technology to particleize acellular dermal matrix in 1999 and launched an AlloDerm (Cymetra) product in the form of an injectable micronized preparation. In addition, Wright Medical Group developed the Graftjacket product in the form of an injectable preparation and launched the Graftjacket Xpress product, and LNC Bio developed a composition in which hyaluronic acid was cross-linked after particleizing acellular dermal matrix in 2014. However, in the case of the composition in which acellular dermal matrix particles and biocompatible polymers as described above were cross-linked, it had high structural stability, but also had high cross-linking degree, viscoelasticity, hardening degree, and extrusion force, making it suitable for use as fillers and prosthetics, but it was still insufficient for application as a wound dressing. In the case of the micronized form, there was a problem that it could not be fixed to the wound site in a liquid form and flowed out of the dressing, preventing it from being evenly distributed on the wound.

Therefore, there is an urgent need to develop a form of an acellular dermal matrix that can be fixed to the wound site.

### [Prior Art Document]

### [Patent Document]

(Patent Document 1) Korean Patent No. 10-1523878 (2015. 05. 21)

KR 2023 0008422 A discloses a composition using a fibrotic acellular dermal matrix, wherein the composition is manufactured without adding a biocompatible polymer, and a method for manufacturing the same.

US 2010/272782 A1 discloses tissue repair compositions and methods of making the tissue repair compositions.

### [Disclosure]

### [Technical Problem]

An object of the present disclosure, which has been derived to solve the problems described above, is to provide a composition including a drying method-applied fibrous acellular dermal matrix which is easy to apply to curved wound sites and has enhanced physical properties by applying a process of drying in a mold without applying a biocompatible polymer, and a method for manufacturing the same.

### [Technical Solution]

In order to achieve the above object, the present invention provides a composition according to claim 1 and a method according to claim 5. Further advantageous embodiments of the present invention are disclosed in the dependent claims.

### [Advantageous Effects]

According to one embodiment of the present disclosure, since a composition including a drying method-applied fibrous acellular dermal matrix and a method for preparing the same do not have a biocompatible polymer added thereto, there is no change in physical properties due to heat, and a step for heat-treating a biocompatible polymer is not required, so the preparation process can be simplified and the preparation cost can be reduced.

In addition, since it has a structure very similar to the dermal matrix of a wound site of a human body, it has excellent bioadhesiveness and preservability in the body, and since a moist environment is well maintained, the treatment effect is excellent and it can be easily applied to a curved wound site.

In addition, the physical properties and tensile strength are improved by performing a step of drying in a mold, so that the operator can use it more easily during the procedure.

### [Description of Drawings]

FIG. 1 is a process diagram for explaining the method for preparing a composition including a drying method-applied fibrous acellular dermal matrix according to another embodiment of the present disclosure.
FIG. 2 is a photograph showing weights of the composition including a fibrous acellular dermal matrix according to Example 1 before and after high-temperature drying treatment.
FIG. 3 is a photograph showing the compositions including a drying method-applied fibrous acellular dermal matrix, prepared in Example 1 and Comparative Examples 1 to 3.
FIG. 4 is a graph showing the results of Experimental Example 2.
FIG. 5 is photographs showing the results of Experimental Example 3.
FIG. 6 is a graph showing the results of Experimental Example 4.

### [Mode for Disclosure]

Hereinafter, the present disclosure will be described in detail with reference to the attached drawings as embodiments of the present disclosure. However, the following embodiments are presented as examples of the present disclosure, and if it is judged that a specific description of a well-known technology or configuration to those skilled in the art may unnecessarily obscure the gist of the present disclosure, the detailed description thereof may be omitted, and the present disclosure is not limited thereby. The present disclosure can be variously modified and applied within the description of the patent claims described later and the equivalent scope interpreted therefrom.

In addition, the terminologies used in this specification are terminologies used to appropriately express the preferred embodiments of the present disclosure, and these may vary depending on the intention of the user or operator or the customs of the field to which the present disclosure belongs. Therefore, the definition of these terminologies should be made based on the contents throughout this specification. Throughout the specification, when a part is said to "include" a certain component, this does not mean that, unless otherwise specifically stated, other components are excluded, but other components may be further included.

Throughout this specification, '%' used to indicate the concentration of a specific substance, unless otherwise stated, is %(w/w) for solid/solid, %(w/v) for solid/liquid, and %(v/v) for liquid/liquid.

Hereinafter, a composition including a fibrous acellular dermal matrix according to one embodiment of the present disclosure will be described in detail.

The composition including a fibrous acellular dermal matrix of this embodiment includes a fibrous acellular dermal matrix and water.

A fibrous acellular dermal matrix (ADM) is a component widely used for the purpose of reconstruction and regeneration, and reinforcement of skin, tendons, and ligaments in plastic surgery and orthopedics by having excellent biocompatibility due to high cell adhesion ability and low immune response compared to existing animal-derived products. The fibrous acellular dermal matrix used in this embodiment may include 90% or more of ones with a long axis length of 10 to 3,000 µm, preferably 100 to 2,000 µm, and more preferably 50 to 90% of ones with a long axis length of 200 to 800 µm. However, if a large number of the fibrous acellular dermal matrixes with a long axis length of less than 100 µm are included, the form may not be properly formed when they are to be processed into a sheet type, or it may be formed only in a film form, making it difficult to flexibly adhere to a wound site. Such a fibrous acellular dermal matrix may be contained in an amount of 48 to 95.5% by weight.

Water is a component added for rehydration, and may be contained in an amount of 0.5 to 52% by weight. If the amount of water is less than 0.5% by weight, it may be difficult to hydrate the acellular dermal matrix, and if it exceeds 52% by weight, it may be difficult to implement the desired formulation due to excessive use.

In this embodiment, since a biocompatible polymer is not added, and thus there is no change in the physical properties due to heat, and a step for heat-treating a biocompatible polymer is not required, the preparation process can be simplified and the preparation cost can be reduced. In addition, since it has a structure very similar to the dermal matrix of a wound site of a human body, it has excellent bioadhesiveness and preservability in the body, and it is good at maintaining a moist environment, so the treatment effect is excellent and it can be easily applied to a curved wound site.

In this embodiment, the fibrous acellular dermal matrix may mean a particleized acellular dermal matrix in the form of a thin and long fiber like a thread rather than a spherical or streamlined individual shape.

In this embodiment, the composition may further include a stem cell, a growth factor, or a mixture thereof.

In addition, in this embodiment, the composition may further include one or more selected from an antibacterial agent, an excipient, and an additive which are used pharmaceutically.

The antibacterial agent may include one or more selected from short-chain alcohols, benzalkonium chloride (BAC), didecyl dimethyl ammonium chloride (DDAC), zeolites (CWT-A), isothiazolones, alkyl dimethyl ammonium chlorides, triazines, 2-(thiocyanato-methylthio)benzothiazole, methylene bis(thiocyanate), acrolein, dodecyl guanidine hydrochloride, chlorophenols, quaternary ammonium salts, glutaraldehyde, dithiocarbamate, 2-mercaptobenzothiazole, para-chloro-meta-xylenol, silver, chlorhexidine, polyhexamethylene biguanide, n-halamines, triclosan, phospholipids, alpha hydroxy acids, 2,2-dibromo-3-nitrilopropionamide, 2-bromo-2-nitro-1,3-propanediol, farnesol, iodine, bromine, hydrogen peroxide, chlorine dioxide, vegetable oil, plant extract, benzalkonium chloride, chlorine, and sodium hypochlorite.

The excipient may include one or more selected from stabilizers, antioxidants, osmotic pressure-adjusting agents, buffers, and pH-adjusting agents, and specifically, may include one or more selected from starch, cellulose, glucose, lactose, sucrose, gelatin, corn, rice, wheat flour, chalk, silica gel, magnesium stearate, sodium stearate, glycerol monostearate, sodium chloride, glycerol, propylene glycol, water, and ethanol.

The additive may include one or more selected from physiologically biocompatible buffers (tromethamine hydrochloride), chelating agents (DTPA or DTPA-bisamide), and calcium chelating complexes (calcium DTPA, CaNaDTPA-bisamide), and optionally, a calcium or sodium salt (calcium chloride, calcium ascorbate, calcium gluconate, or calcium lactate).

The composition may further include one or more of a stem cell and a growth factor.

In this embodiment, the composition including the fibrous acellular dermal matrix may be prepared in the form of an aqueous solution, a suspension, an emulsion, a paste, a cream, a balm, an ointment, a foam, a sheet, a gel, a gum, a spray, a slurry, a film, a granule, a patch, a powder, etc., but may be a desirable sheet type, and may be used as a dressing, an adhesive, a surgical and medical device, an artificial skin, a bandage, a foaming agent, an anti-adsorption agent, or a grafting material.

Hereinafter, a method for preparing a composition including a fibrous acellular dermal matrix according to another embodiment of the present disclosure will be described in detail with reference to the drawings.

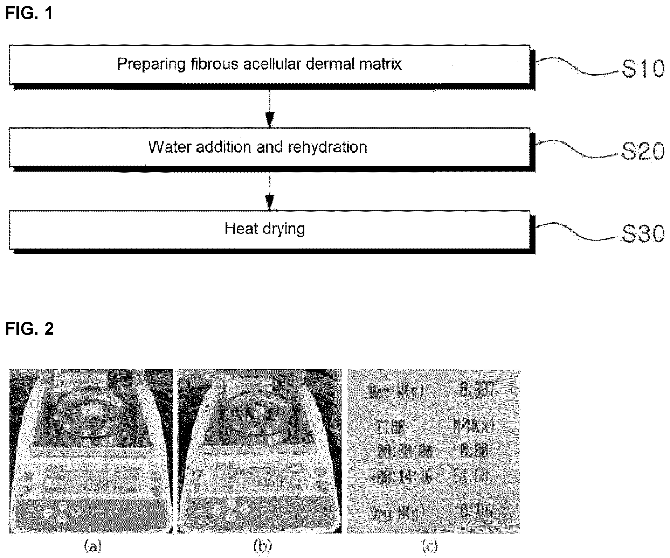

FIG. 1 is a process diagram for explaining the method for preparing a composition including a drying method-applied fibrous acellular dermal matrix according to another embodiment of the present disclosure.

Referring to Fig. 1, first, an acellular dermal matrix is pulverized to prepare a fibrous acellular dermal matrix (S10).

The acellular dermal matrix may be pulverized by one or more pulverizers selected from a cutting mill, a food processor, a mano pulverizer, a freeze pulverizer, a micronizer, a vibratory micro mill, a jaw crusher, a mortar grinder, a planetary mill, a disk mill, a ball mill, a knife mill, and a variable speed rotor mill to prepare a fibrous acellular dermal matrix. At this time, it is preferable to set the pulverizer to a rotation speed of 500 to 2000 rpm so that pulverization is easily performed in a short period of time.

Meanwhile, in this embodiment, the acellular dermal matrix may include one prepared by steps of preparing human body-derived skin tissue that has not undergone a separate de-epidermization and de-adipization process, treating the skin tissue with a storage solution containing a surfactant, and washing the treatment-completed skin tissue with an isotonic solution, wherein it is preferable to apply one having a thickness of 0.1 mm to 3 mm as the acellular dermal matrix.

Next, water is added to the fibrous acellular dermal matrix to rehydrate it (S20).

300 to 2,000 parts by weight of water may be added to 100 parts by weight of the acellular dermal matrix prepared in the above step S10 to hydrate it. If the water content is less than 300 parts by weight, it may be difficult to hydrate the fibrous acellular dermal matrix, and if it exceeds 2,000 parts by weight, it may be undesirable since the heat drying time described below may be prolonged due to excessive use. In this embodiment, water may be sterilized distilled water.

In addition, the rehydrated fibrous acellular dermal matrix is heat-dried (S30).

In the above step S20, the rehydrated fibrous acellular dermal matrix may be heat-dried in a mold, preferably a lattice-shaped mold, at 30 to 50°C for 6 hours or more, for example, 10 hours or more, for example, 10 to 24 hours, preferably 16 to 18 hours, until the moisture content becomes 0.5 to 52% by weight. If the temperature is less than 30°C, excessive time may be required for drying, and if it exceeds 50°C, human body tissue may be deformed, so it may not only reduce the amino acid content, but also reduce the tensile strength and hydration degree.

As described above, the average tensile strength value of the fibrous acellular dermal matrix satisfies 0.2 N/mm² or more by the heat-drying conditions, so that the operator can use it more easily than before during the procedure.

The method may further include a step of processing the heat-dried fibrous acellular dermal matrix into a sheet type after the step S30.

In the step S30, it goes without saying that the heat-dried fibrous acellular dermal matrix may be applied to a mold (or frame) and then shaped and processed into a sheet type, and it may be prepared in various ways depending on the width, length, and thickness sizes required by the wound site or surgical characteristics.

Meanwhile, prior to processing it into a sheet type, a non-chemical crosslinking process using radiation irradiation, decompression, and heat or a chemical crosslinking process using a crosslinking agent may be additionally performed depending on the physical properties (crosslinking degree, degree of curing, etc.).

Hereinafter, the present disclosure will be described in more detail using Examples. These Examples are only intended to more specifically illustrate the present disclosure, and it will be apparent to those skilled in the art that the scope of the present disclosure is not limited by them.

### Preparation Example 1. Preparation of acellular dermal matrix

Skin tissues were purchased from EURO skin bank, Allosource, and CTS, and tissues with a thickness of 0.5 mm or more were selected, and fat tissues attached to the skin tissues were removed using forceps, and then washed three times with sterilized water. Then, the tissues were immersed in a hypotonic solution (Tris-HCl, EDTA, NaOH, and SDS) and treated for 6 hours. In addition, the skin tissues were washed with PBS at 4°C to remove fat, epidermis, cells, and hypotonic solution remaining in the skin tissues, and left overnight. Then, the skin tissues were washed with an isotonic solution (Tris-HCl, EDTA, NaCl, NaOH) for 6 hours to prepare a human body-derived acellular dermal matrix that was de-epidermized, defatted, and decellularized.

### Example 1. Preparation of a composition including a fibrous acellular dermal matrix

The human body-derived acellular dermal matrix prepared in the above Preparation Example 1 was pulverized with a cutting mill and fibrosed to prepare a fibrous acellular dermal matrix. 10 times of sterilized distilled water was added thereto to rehydrate it, and after it was placed in a grid-shaped mold and heat-dried at 30°C to 50°C for more than 6 hours, a composition including a fibrous acellular dermal matrix was prepared by applying it to a mold to shape it and processing it into a sheet type.

Meanwhile, the weights before and after heat-drying treatment for 10 minutes or more at a high temperature condition of 126°C for the composition including a fibrous acellular dermal matrix according to Example 1 are shown in FIG. 2.

When checking FIG. 2(C), it can be confirmed that the composition including a fibrous acellular dermal matrix includes 48.32% by weight of a fibrous acellular dermal matrix and 51.68% by weight of water from the weight before drying of 0.387 g shown in FIG. 2(A) and the weight after drying of 0.187 g shown in FIG. 2(B).

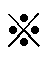 In Example 1, drying was not performed overnight at RT which is a temperature of less than 30°C.

### Comparative Example 1. Preparation of a composition including a fibrous acellular dermal matrix

It was prepared in the same manner as in Example 1 except that the heat treatment was performed at a 60°C condition for 6 hours or more.

### Comparative Example 2. Preparation of a composition including a fibrous acellular dermal matrix

It was prepared in the same manner as in Example 1 except that the heat treatment was performed at a 70°C condition for 6 hours or more.

### Comparative Example 3. Preparation of a composition including a fibrous acellular dermal matrix

It was prepared in the same manner as in Example 1 except that the heat treatment was performed at a 100°C condition for 6 hours or more.

### Experimental Example 1. Appearance evaluation

The appearances of the compositions including a fibrous acellular dermal matrix prepared in Example 1 and Comparative Examples 1 to 3 were visually checked and evaluated.

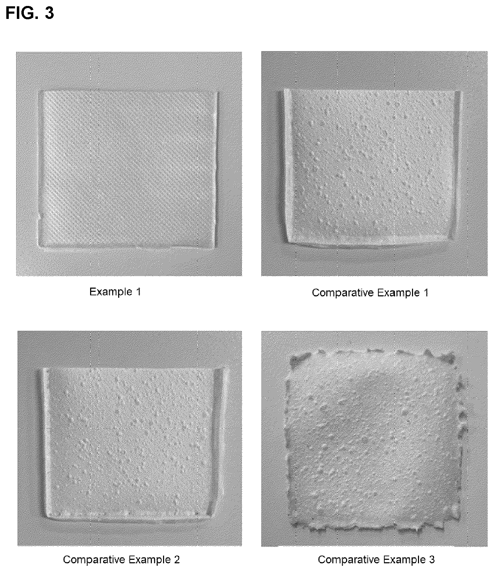

As results, as shown in FIG. 3, it could be confirmed that Example 1 had a structure very similar to the dermal matrix of a wound site of a human body in terms of appearance, Comparative Examples 1 and 2 showed some differences from the dermal matrix of the wound site of the human body in terms of appearance, and Comparative Example 3 had a burnt-out tissue.

In this way, it can be confirmed that the composition including a fibrous acellular dermal matrix of the present disclosure does not need to be heated at a high temperature since no biocompatible polymer is added, so there is no change in physical properties due to heat, and since a step for heat-treating a biocompatible polymer is not required, the preparation process can be simplified and the preparation cost can be reduced.

### Experimental Example 2. Evaluation of amino acid content depending on heat treatment temperature

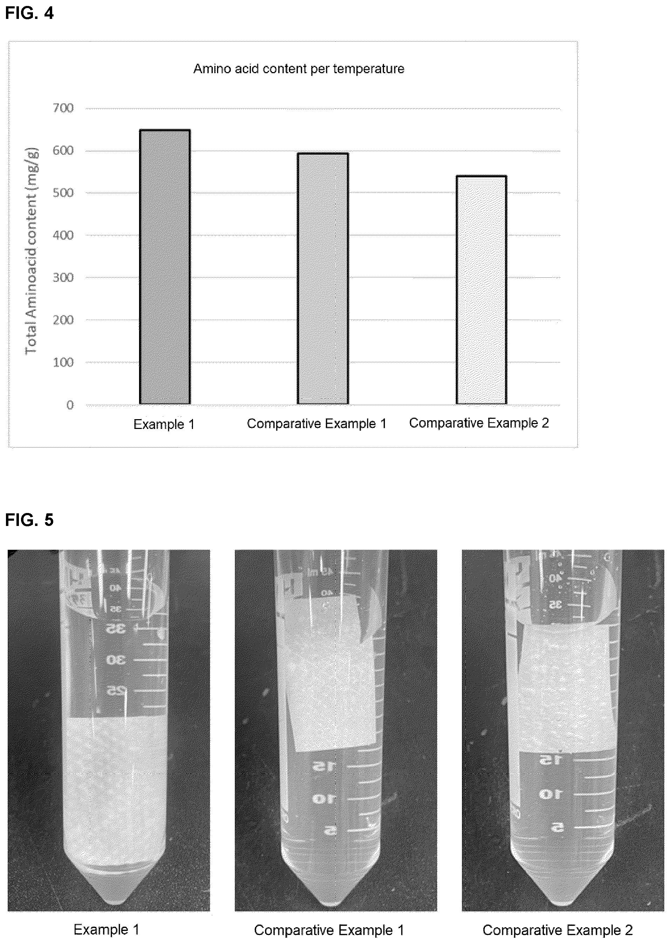

In order to confirm the change in amino acid content depending on heat treatment temperature, the amino acid contents of Example 1 and Comparative Examples 1 and 2 were measured using an amino acid auto-analyzer (S433D, Sykam GmbH Co., Germany), and the results are shown in FIG. 4.

Referring to FIG. 4, it could be confirmed that the amino acid content decreased as it went from Example 1 to Comparative Examples 1 and 2, and it was expected that this was because human body tissue deformation occurred in Comparative Examples 1 and 2.

In this way, it can be seen that the composition including a fibrous acellular dermal matrix prepared according to the Example of the present disclosure has a structure very similar to the dermal matrix of a wound site of a human body, and thus has excellent bioadhesiveness and preservability in the body, and thus has an improved therapeutic effect and is easy to apply to a curved wound site.

### Experimental Example 3. Evaluation of hydration degree depending on heat treatment temperature

In order to check the hydration degree depending on heat treatment temperature, Example 1 and Comparative Examples 1 and 2 were placed in a tube containing water and observed, and the results are shown in FIG. 5.

Referring to FIG. 5, Example 1 sank within a few seconds, but it could be confirmed that Comparative Examples 1 and 2 did not sink but continued to float, which was expected to be due to the effects of drying and shrinkage due to high temperatures.

In this way, it can be seen that the composition including a fibrous acellular dermal matrix prepared according to the Examples of the present disclosure has an excellent therapeutic effect by maintaining a moist environment well and is easy to apply to a curved wound site.

### Experimental Example 4. Evaluation of tensile strength depending on heat treatment temperature

In order to check the tensile strengths depending on the heat treatment temperatures, the results are shown in FIG. 5.

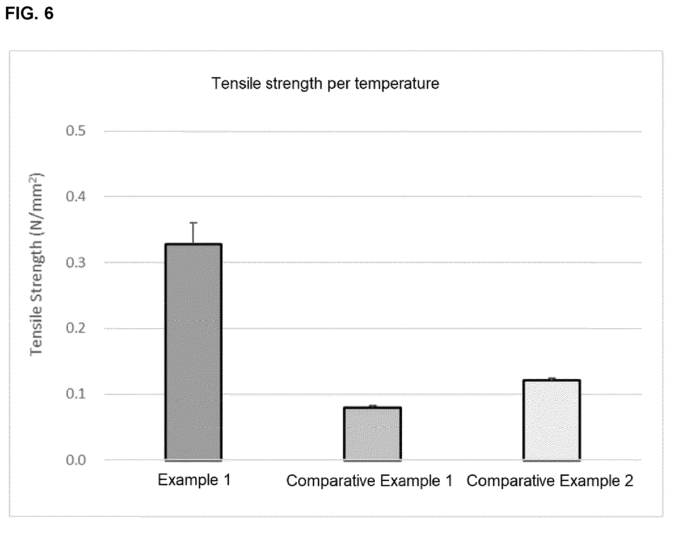

Referring to FIG. 6, it could be confirmed that the tensile strengths of Comparative Examples 1 and 2 decreased compared to that of Example 1.

In this way, it can be seen that the composition including a fibrous acellular dermal matrix prepared according to the Example of the present disclosure does not need to be heated at a high temperature since no biocompatible polymer is added, so there is no change in the physical properties due to heat, and the physical properties and tensile strength are improved by performing heat drying, especially the step of performing heat drying in a mold, so that it is easy for the practitioner to use it during the procedure.

Although the exemplary embodiments of the present disclosure have been described in detail above, the scope of rights of the present disclosure is not limited thereto, and various modifications and improved forms made by those skilled in the art using the basic concept of the present disclosure defined in the following claims also fall within the scope of rights of the present disclosure.

All technical terms used in the present disclosure, unless otherwise defined, are used with the same meaning as generally understood by those skilled in the art in the relevant field of the present disclosure. The contents of all publications described as reference documents in this specification are introduced into the present disclosure.

## Claims

1. A composition comprising a fibrous acellular dermal matrix, the composition comprising:
48 to 95.5% by weight of a fibrous acellular dermal matrix; and
0.5 to 52% by weight of water,
wherein the composition has no biocompatible polymer added thereto, and
wherein the fibrous acellular dermal matrix satisfies an average tensile strength value of 0.2 N/mm² or more.

2. The composition of claim 1, further comprising a stem cell, a growth factor, or a mixture thereof.

3. The composition of claim 1 or 2, wherein the composition is a sheet type.

4. The composition of any one of claims 1 to 3, wherein the composition is prepared by the method of any one of claims 5 to 7.

5. A method for preparing a composition comprising a fibrous acellular dermal matrix that satisfies an average tensile strength value of 0.2 N/mm² or more, the method comprising steps of:
(a) pulverizing an acellular dermal matrix to prepare a fibrous acellular dermal matrix;
(b) adding water to the fibrous acellular dermal matrix to rehydrate it; and
(c) heat-drying the rehydrated fibrous acellular dermal matrix,
wherein the heat-drying in the step (c) is performed at 30 to 50°C for 6 hours or more.

6. The method of claim 5, wherein the heat-drying in the step (c) is performed in a mold.

7. The method of claim 5 or 6, further comprising a step (d) of processing the heat-dried fibrous acellular dermal matrix into a sheet type after the step (c).

## Patentansprüche

1. Eine Zusammensetzung, die eine fibröse azelluläre dermale Matrix aufweist, wobei die Zusammensetzung aufweist:
48 bis 95,5 Gew.-% einer fibrösen azellulären dermalen Matrix und
0,5 bis 52 Gew.-% Wasser,
wobei der Zusammensetzung kein biokompatibles Polymer zugesetzt ist und
wobei die fibröse azelluläre dermale Matrix einen durchschnittlichen Zugfestigkeitswert von 0,2 N/mm²oder mehr erfüllt.

2. Die Zusammensetzung gemäß Anspruch 1, die ferner eine Stammzelle, einen Wachstumsfaktor oder eine Mischung davon aufweist.

3. Die Zusammensetzung gemäß Anspruch 1 oder 2, wobei die Zusammensetzung ein Folientyp ist.

4. Die Zusammensetzung gemäß irgendeinem der Ansprüche 1 bis 3, wobei die Zusammensetzung mittels des Verfahren gemäß einem der Ansprüche 5 bis 7 hergestellt ist.

5. Ein Verfahren zum Herstellen einer Zusammensetzung, die eine fibröse azelluläre dermale Matrix aufweist, die einen durchschnittlichen Zugfestigkeitswert von 0,2 N/mm²oder mehr erfüllt, wobei das Verfahren die Schritte aufweist des:
(a) Pulverisierens einer azellulären dermalen Matrix, um eine fibröse azelluläre dermale Matrix herzustellen,
(b) Zugebens von Wasser zu der fibrösen azellulären dermalen Matrix, um diese zu rehydrieren, und
(c) Wärmetrocknens der rehydrierten fibrösen azellulären dermalen Matrix,
wobei das Wärmetrocknen in Schritt (c) bei 30 bis 50°C für 6 Stunden oder mehr durchgeführt wird.

6. Das Verfahren gemäß Anspruch 5, wobei das Wärmetrocknen in Schritt (c) in einer Form durchgeführt wird.

7. Das Verfahren gemäß Anspruch 5 oder 6, das ferner einen Schritt (d) des Verarbeitens der wärmegetrockneten fibrösen azellulären dermalen Matrix in einen Folientyp nach dem Schritt (c) aufweist.

## Revendications

1. Composition comprenant une matrice dermique acellulaire fibreuse, ladite composition comprenant :
48 à 95,5 % en poids d'une matrice dermique acellulaire fibreuse ; et
0,5 à 52 % en poids d'eau,
dans laquelle aucun polymère biocompatible n'est ajouté à la composition, et
dans laquelle la matrice dermique acellulaire fibreuse satisfait à une valeur de résistance à la traction moyenne de 0,2 N/mm² ou plus.

2. Composition selon la revendication 1, comprenant en outre une cellule souche, un facteur de croissance ou un mélange de ceux-ci.

3. Composition selon la revendication 1 ou 2, dans laquelle la composition est d'un type de feuille.

4. Composition selon l'une quelconque des revendications 1 à 3, dans laquelle la composition est préparée moyennant le procédé selon l'une quelconque des revendications 5 à 7.

5. Procédé de préparation d'une composition comprenant une matrice dermique acellulaire fibreuse qui satisfait à une valeur de résistance à la traction moyenne de 0,2 N/mm² ou plus, le procédé comprenant les étapes suivantes consistant à :
(a) pulvériser une matrice dermique acellulaire pour préparer une matrice dermique acellulaire fibreuse ;
(b) ajouter de l'eau à la matrice dermique acellulaire fibreuse afin de la réhydrater ; et
(c) sécher à la chaleur la matrice dermique acellulaire fibreuse réhydratée,
dans lequel le séchage à la chaleur dans l'étape (c) est effectué à 30 à 50 °C pendant 6 heures ou plus.

6. Procédé selon la revendication 5, dans lequel le séchage à la chaleur dans l'étape (c) est effectué dans un moule.

7. Procédé selon la revendication 5 ou 6, comprenant en outre une étape (d) consistant à transformer la matrice dermique acellulaire fibreuse séchée à la chaleur en un type de feuille après l'étape (c).
